# EUROPEAN PATENT APPLICATION

(11) **EP 2 218 474 A1**
(43) Date of publication of application: **18.08.2010**
(21) Application number: 10153029.3
(22) Date of filing: 09.02.2010
(51) Int. Cl.: A61M 25/01, A61B 19/00, A61B 17/34

(54) **Orientable device for the support of an adaptor and introductor of catheters in the human cardiovascular system**

(30) Priority: 11.02.2009 IT BO20090005 U
(71) Applicant: Tre Esse Progettazione Biomedica S.r.l, 40138 Bologna (IT)
(72) Inventor: Plicchi, Gianni, I-40125, BOLOGNA (IT); Marcelli, Emanuela, I-40125, BOLOGNA (IT); Cercenelli, Laura, I-60030, CASTELBELLINO, Province of Ancona (IT); Panfili, Mauro, I-40139, BOLOGNA (IT)
(74) Representative: Porsia, Dino

(57) **Abstract**

Swivel device for the adjustable and removable support of an adapter and introducer unit (A) which facilitates the temporary insertion of flexible catheters (E) into the human cardiovascular system. Part of the body of said unit (A) is provided with an annular channel or other suitable means (1) by which it can be fitted and retained both axially and radially in a fork (2) mounted on a sliding block (4) which can be made to slide in a guide (5) between a screw and nut unit (6) which can be driven by means of a knob (7) or by a remote control system, said guide (5) being mounted on the end of any suitable hinged and/or jointed arm (8) which can be locked in a predetermined position and which carries on its other end means (9) of the clamp or other type which can be fixed to the frame (110) of the bed of other structure supporting the patient (P), the whole arrangement being such that said adapter and introducer unit (A) can be supported steadily by the device in question, with the possibility of simple orientation and adjustment in respect of distance from the patient and with the possibility of simple and rapid detachment of the device.

## Description

The invention relates to a swivel device for the adjustable and removable support of an adapter and introducer unit which facilitates the temporary insertion of flexible catheters, for diagnostic and/or therapeutic purposes, into the human cardiovascular system, and also relates to the improvements made to the adaptor and introducer unit to make it suitable for interaction with the device in question.

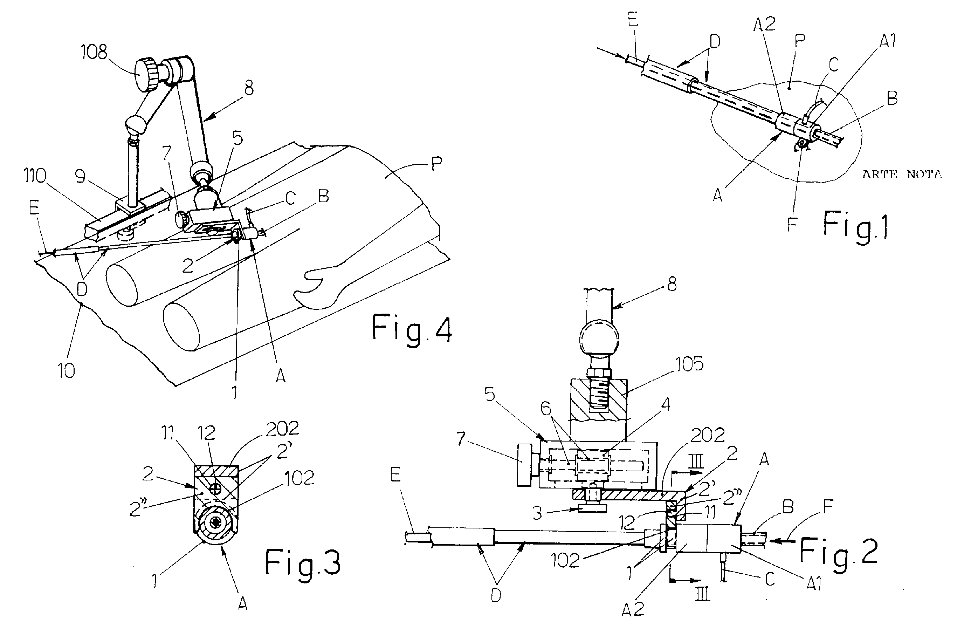

The adapter and introducer unit to which reference is made is of the type which is described in Italian patent application BO2005A-798 in the name of the present applicant and which is schematically illustrated in Figure 1 of the attached drawings, comprising a composite cylindrical body A, formed by an introducer A1 having at one end a cannula B which is inserted into an artery or vein of the patient P and which comprises a lateral tube C for the admission of anticoagulant lubricating liquid into said cannula B, and by a rear adapter A2 whose free end is designed to be coupled to a telescopic guide D which keeps the catheter E rigid when it is pushed axially through the guide D and through the adapter and introducer unit A for insertion into the patient's cardiovascular system. The front introducer A1 of the unit A has an inner sphincter seal which is normally closed to isolate the patient's vein or artery from the outside, while the upstream adapter A2 has a ring seal correlated with the diameter of the catheter E and has a front spreader point which is inserted into said sphincter seal of the introducer to expand it and thus allow the catheter to advance axially while meeting only the resistance of said calibrated seal of the adapter, which is minimal, thus facilitating the axial advance and retraction movements of the catheter E.

The body of said unit A, usually that of the introducer A1, is at present provided with a lateral slotted lug F which can be fixed to the patient's skin with a suture, as shown in Figure 1.

It has been found that, in order to facilitate the advance and steering of the tip of the catheter during the catheter movement phase, it is necessary to provide a steady and secure support for said adapter and introducer unit A with the upstream telescopic guide D, in order to be able to modify the orientation and/or distance of said set of apparatus with respect to the patient's body.

The invention is intended to overcome these problems of the prior art with a device of simplified construction and of high reliability and safety, as described in the attached Claim 1 and the subsequent dependent claims, based on the following proposed solution. The body of said unit A, for example that of the adapter A2, is provided with an annular channel such that it can be fitted in a fork mounted on a sliding block which can be made to travel in a guide by means of a screw and nut unit which can be operated by means of a knob or by a remote control system. The unit formed by the guide and the sliding block with said fork is mounted on the end of any suitable hinged and/or jointed arm which can be locked in a predetermined position and which carries on its other end means, of the clamp or other type, which can be fixed to the frame of the bed or other structure supporting the patient.

Further characteristics of the invention, and the advantages derived therefrom, are illustrated more fully in the following description which refers to the figures on the single attached sheet of drawing, in which, in addition to Figure 1 which has already been discussed,
- Fig. 2 shows the operating part of the device, with parts shown in cross section, in the condition in which it is interacting with the adapter and introducer unit;
- Fig. 3 shows further details of the device in a view taken through the section line III-III of Figure 2;
- Fig. 4 is a schematic perspective illustration of the device in use on a patient.

Figures 2 and 3 show how the body of the adapter A2 of the unit A, usually made of plastic material, is provided with an annular groove 1 by means of which the adapter can be friction fitted (see below) into the channel 102, which is suitably curved and has rounded flared ends, of a control fork 2. It is to be understood that the scope of the invention includes the variant in which the fork 2 can be designed to interact with a projecting part, instead of a recessed part, of the body of the adapter A2. The fork 2 has a right-angled shank 202 which is parallel to the axis of the unit A, and is designed to be fixed by any suitable means 3 to a sliding block 4 which can slide in a rectilinear guide 5 and can be driven therein by a screw and nut unit 6, the screw being rotatable in both directions, for example by a knob 7 or by other suitable means, for example by remote control means, or by mechanical means, such as sheathed cable means, or by electromechanical means, such as a small geared motor and encoder, or by other means.

The sliding block 5 has a shank 105 in a position preferably opposite that of the fork 2, this shank being fastenable to the jointed end of a hinged and/or jointed arm 8 of any type including commercially available types, for example a NOGA ® arm, the other end of which has a clamp 9 or any suitable means such that it can be fixed rapidly and removably, for example to the frame 110 of the bed or any other structure 10 supporting the patient P. The aforesaid arm 8 is such that all the hinges and joints of the arm can be released and locked by means of its intermediate knob 108.

Figure 4 shows how the device according to the invention enables the unit A and the telescopic guide D to be supported steadily in any arrangement and in any orientation and configuration with respect to the patient P, and how the operating device 7 can be used to adjust precisely the distance of the unit A and D from the patient, to facilitate the insertion of the catheter E into the patient's cardiovascular system for use. The unit A and D can be released from the fork 2 and released from the support device at any time in order to permit manual operation.

To ensure that any unforeseen changes in the posture of the patient, who may be seated, do not cause injury to the patient because of an excessively rigid coupling of the unit A to the support and control device proposed by the invention, the unit A is made to be automatically releasable from the fork 2 of the device if the unit A is subjected to an axial force F, as shown in Figure 2, which exceeds a predetermined safe level, for example about 100 g. This object is achieved by means of a fork 2 which, as shown in Figures 2 and 3, comprises a part 2" with the channel 102 for coupling to the unit A, linked to the rear of the part 2' of the fork by means of a snap fastener 11-12 of the mechanical type as in the illustrated example, or of another type, such as a magnetic type. The device which is described is suitable for use and installation in combination with external protective means, formed for example by a sterile disposable transparent plastic bag, which is not shown herein but which is such that it permits frictional interaction between the fork 2 and the channel 1 of the adapter and introducer unit A, the fixing of the clamp 9, and the convenient operation of the knob 7.

It is to be understood that the device described herein can be varied and modified in numerous ways in respect of its construction, for example in respect of the fork 2 which can be constructed differently in the form of a resilient or sprung gripper and/or which can be provided with an easily closed and opened loop or collar to retain the unit A, possibly without the use of friction and without requiring push fitting which may cause undesired movements of the unit A with respect to the patient, all such modifications being possible without departure from the guiding principle of the invention as described and illustrated and as claimed below.

## Claims

1. Swivel device for the adjustable and removable support of an adapter and introducer unit (A) which facilitates the temporary insertion of flexible catheters (E) into the human cardiovascular system, **characterized in that** part of the body of said unit (A) is provided with an annular channel or other suitable means (1) by which it can be fitted and retained both axially and radially in a fork (2) mounted on a sliding block (4) which can be made to slide in a guide (5) between a screw and nut unit (6) which can be driven by means of a knob (7) or by a remote control system, said guide (5) being mounted on the end of any suitable hinged and/or jointed arm (8) which can be locked in a predetermined position and which carries on its other end means (9) of the clamp or other type which can be fixed to the frame (110) of the bed of other structure supporting the patient (P), the whole arrangement being such that said adapter and introducer unit (A) can be supported steadily by the device in question, with the possibility of simple orientation and adjustment in respect of distance from the patient and with the possibility of simple and rapid detachment of the device.

2. Device according to Claim 1, **characterized in that** said fork (2) is made from at least two parts (2', 2") joined together by a snap fastener (11, 12) or other suitable means having a predetermined coupling force, said two parts of the fork being disconnected by a force in excess of this coupling force and thereby detaching the adapter and introducer unit (A) from the support device in question, to ensure the safety of the patient, for example in case of accidental changes in posture.

3. Device according to Claim 1, **characterized by** the use of a hinged and/or jointed arm (8) with a rapid and simplified locking and release system, for example an arm of the type marketed under the trade name NOGA®.

4. Device according to the preceding claims, in which said fork (2) can be made in the form of a resilient or oscillating fork or gripper and/or can be provided with a closing and opening loop or collar to retain the adapter (A), possibly without the use of friction and without the need to exert any force or pressure on the adapter.

5. Device according to the preceding claims, **characterized in that** it can be used and installed in combination with external protective means, formed for example by a sterile disposable transparent plastic bag which is such that it permits interaction between the fork (2) and the channel (1) of the adapter and introducer unit (A), the fixing of the clamp (9) of said arm (8), and the operation of the knob or other means of adjustment (7).
